(19) Europäisches Patentamt
European Patent Office
Office européen des brevets

(11) Publication number: 0 367 599 A1

(12) # EUROPEAN PATENT APPLICATION

(21) Application number: 89311339.9

(51) Int. Cl.5: C07D 207/44 , C07D 403/10

(22) Date of filing: 02.11.89

(30) Priority: 03.11.88 GB 8825790

(43) Date of publication of application:
09.05.90 Bulletin 90/19

(84) Designated Contracting States:
AT BE CH DE ES FR GB GR IT LI LU NL SE

(71) Applicant: BP Chemicals Limited
Belgrave House 76 Buckingham Palace Road
London, SW1W 0SU(GB)

(72) Inventor: Lancaster, Michael BP Chem.
Limited Barry Factory
Hayes Road Sully Penarth
South Glamorgan CF6 2YU Wales(GB)

(74) Representative: Krishnan, Suryanarayana
Kalyana et al
BP INTERNATIONAL LIMITED Patents &
Agreements Division Chertsey Road
Sunbury-on-Thames Middlesex TW16
7LN(GB)

(54) **Synthesis of bisimides.**

(57) The present invention relates to a process for producing bisimides of unsaturated cyclic dianhydrides by cyclodehydration of the corresponding bisamic acid using acetic anhydride and a base component characterised in that the base component is free of amino compounds and comprises a carbonate or bicarbonate of an alkali metal or an alkaline earth metal.

EP 0 367 599 A1

## SYNTHESIS OF BISIMIDES

This invention relates to a process for the synthesis of bisimides of unsaturated cyclic dianhydrides especially bismaleimides in the presence of sodium acetate catalysed by metal salts.

Bisimides of unsaturated cyclic dianhydrides such as bismaleimides are useful crosslinking agents for elastomers and in the copolymerisation of vinyl monomers such as styrene. For instance copolymers of bismaleimides with other monomers result in products of high thermal stability. Bisimides are usually prepared by reacting initially the unsaturated cyclic anhydride with a diamine to form the corresponding bisamic acid which is subsequently cyclodehydrated using acetic anhydride and a salt such as sodium acetate to form the bisimide. Such procedures for producing 2,4-bis(maleimido) toluene are described in DT-A-2127025 (Techno-Chemie GmbH), US-A-2444536 (DuPont) and US-A-3127414 (DuPont).

USP 4154737 (DuPont) describes such a process to have serious disadvantages due to the product isolation stages requiring large amounts of water to precipitate the end product and the conversion of the expensive dimethylformamide (DMF) solvent by acetic acid used in the reaction into wasteful dimethyl acetamide and formic acid. This DuPont patent recommends the use of a combination of salts of magnesium, lithium, manganese or cobalt which are soluble in the liquid reaction medium and a cocatalyst which is a tertiary amine having a pKa value of at least 10 determined in aqueous solution at 25° C.

This combination used instead of sodium acetate is said to mitigate the problems of the latter.

US-A-3839358 (Rhone-Poulenc) describes a process for preparing bismaleimides by using in the cyclodehydration step bismaleamic acid, a liquid lower carboxylic acid anhydride, a tertiary amine and a catalyst which is a nickel salt or a divalent nickel complex. Similar processes are also described in US-A-3975401 and US-A-3960887 both to Rhone-Poulenc.

It has now been found that the cyclodehydration of the bisamic acid can be achieved rapidly at relatively low temperatures and in high yield in the presence of an anhydride and a carbonic acid salt.

Accordingly, the present invention is a process for producing bisimides of unsaturated cyclic dianhydrides by cyclodehydration of the corresponding bisamic acid using acetic anhydride and a base component characterised in that the base component is free of amino compounds and comprises a carbonate or bicarbonate of an alkali metal or an alkaline earth metal.

A typical example of the bisamic acid to be dehydrated is that derived from maleic anhydride and can be represented by the generic formula:

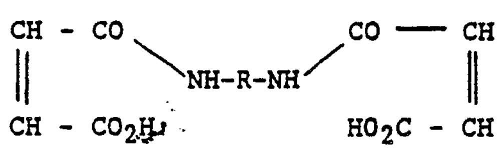

(I)

wherein R is selected from a linear or branched chain $C_2$-$C_{12}$ alkylene group; a cycloalkylene group having six carbon atoms in the ring; an arylene group; and a di-, or poly-alkylene or a di- or poly-arylene group in which the alkylene or arylene groups are respectively bridged by one or more groups selected from:

-$CR_1R_2$-, wherein $R_1$ and $R_2$ may be the same or different groups selected from H, an alkyl, an aryl or an alkaryl group;

-$SO_2$-;

-O-;

-$NR_3$- wherein $R_3$ is an alkyl, aryl or an alkaryl group;

and a -$C(CF_3)_2$- group.

Whilst the above shows bismaleamic acid as a typical example of the bisamic acid, other bisamic acids derivable from corresponding anhydrides such as citraconic and nadic anhydrides instead of maleic anhydride can also be cyclodehydrated by the present process.

The bisamic acid to be cyclodehydrated can be produced by conventional reactions well known to those skilled in the art. For instance, bismaleamic acid can be produced by reacting an appropriate primary amine with maleic anhydride at ambient temperature in the presence of a solvent such as e.g. chloroform, dimethyl formamide, acetone or toluene. Thus

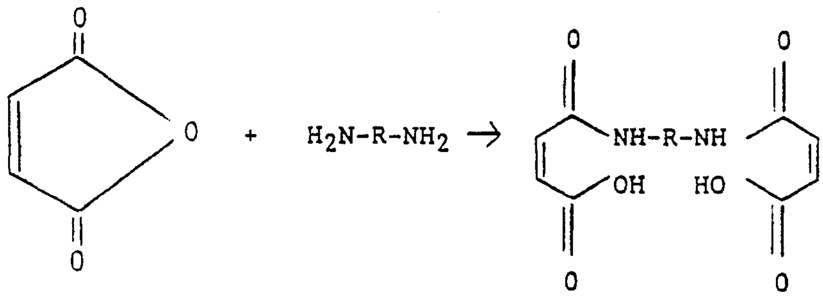

(I)

The bismaleamic acid so formed can be cyclodehydrated to the corresponding bismaleimide as represented below according to the process of the present invention:

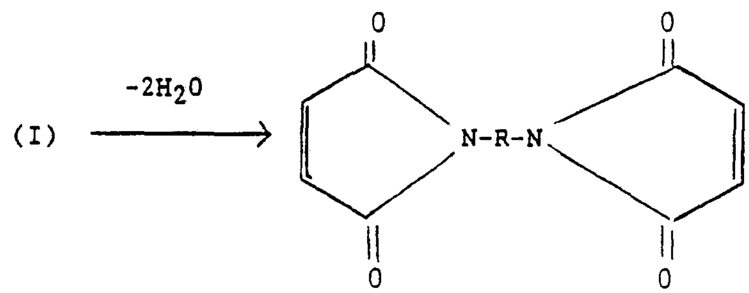


(II)

The cyclodehydration stage according to the present invention is carried out using acetic anhydride and a base component comprising a carbonate or bicarbonate of an alkali metal or an alkaline earth metal. Sodium and potassium carbonates are preferred. The carbonate or bicarbonate used is preferably anhydrous.

The carbonate or bicarbonate is suitably present in the base component in an amount of at least 5% w/w based on the bisamic acid, preferably from 8-15% w/w of the bisamic acid to be cyclodehydrated.

The cyclodehydration stage is optionally carried out in the presence of a catalytic amount of an inorganic salt selected from the acetate, chloride, bromide, nitrate or sulphate salt of potassium; sodium chloride; the chloride or sulphate salt of magnesium; cobalt acetate; and ferric sulphate.

By "catalytic amount" is meant here and throughout the specification that the inorganic salt is present in an amount of at least 0.5% w/w based on the bisamic acid, preferably from 0.5-2% w/w of the bisamic acid to be cyclodehydrated.

The inorganic salt in the base component can be used in the hydrated or anhydrous form. By hydrated is meant that the salt is used with its inherent water of crystallisation intact.

The mole ratio of acetic anhydride to the bisamic acid used for the cyclodehydration is suitably from 2-4 moles of the anhydride, preferably from 2.7-3.2 moles of acetic anhydride per mole of the bisamic acid.

The cyclodehydration is suitably carried out in a solvent medium. The solvent chosen should be such that the reactants and base components are substantially soluble therein. Polar, aprotic solvents such as dimethyl sulphoxide, dimethyl formamide and N-methyl pyrrolidone are preferred.

The cyclodehydration reaction is suitably carried out at a temperature from 20-60 °C, preferably from 30-40 °C.

The reaction mixture is thereafter cooled to room temperature and diluted with water. The bisimide, which is insoluble in water, is precipitated. This product is then filtered, washed and dried.

The present invention is further illustrated with reference to the following Examples.

Example 1

A solution of methylene dianiline (15.2g) in acetone (31g) was added to a solution of maleic anhydride

(15.8 g) in dimethylformamide (62g) over 1 hr. The mixture was stirred at 35° C for 1 hr under a nitrogen atmosphere.

Sodium carbonate (3.1g) was added followed by acetic anhydride (24.3g) over 1/2 hour and during this time the temperature rose to 40° C. The solution was heated at 40° C for 3 hrs before cooling to 25° C. Water ($100cm^3$) was added and the resulting bismaleimide filtered and washed with water. 23g (82%) of methylenedianiline bismaleimide (90% purity by HPLC) were obtained.

A similar procedure using sodium acetate in place of sodium carbonate at 55-60° C for 2 hrs gave a 75% yield of 72% purity. No appreciable conversion to bismaleimide occurred when carrying the reaction out at 40° C for 3 hrs.

Example 2

Preparation of Oxydianiline Bismaleimide

Oxydianiline (27.3g) dissolved in dimethylformamide (137g) was added to a solution of maleic anhydride (27.3g) in acetone (52g) over 1 hr with stirring at ambient temperature. Stirring was continued for a further 1 hr at 30° C after the addition was complete. Acetic anhydride (38.7g) was added over $\frac{1}{2}$ hr followed by sodium carbonate (5.5g) and cobalt acetate (0.6g). An exotherm to 40° C was observed after 10 mins. The mixture was stirred at 40° C for $3\frac{1}{2}$ hrs. After cooling to 25° C water (200 $cm^3$) was added over 1 hr, the product filtered and washed with water and a 1/1 mixture of acetone and methanol and dried. Bismaleimide product (40.4g) (83%) of 95% purity by HPLC was obtained.

A similar procedure using triethylamine as catalyst in place of sodium carbonate gave a 80% yield of bismaleimide of 85% purity.

**Claims**

1. A process for producing bisimides of unsaturated cyclic dianhydrides by cyclodehydration of the corresponding bisamic acid using acetic anhydride and a base component characterised in that the base component is free of amino compounds and comprises a carbonate or bicarbonate of an alkali metal or an alkaline earth metal.

2. A process according to claim 1 wherein the bisamic acid to be dehydrated is that derivable from maleic anhydride and is represented by the generic formula:

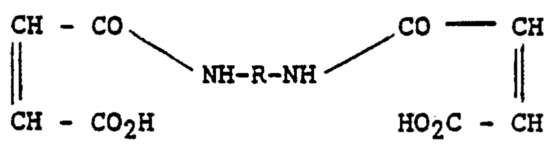

(I)

wherein R is selected from a linear or branched chain $C_2$-$C_{12}$ alkylene group; a cycloalkylene group having six carbon atoms in the ring; an arylene group; and a di- or poly-alkylene or a di- or poly-arylene group in which the alkylene or arylene groups are respectively bridged by one or more groups selected from:
$-CR_1R_2-$, wherein $R_1$ and $R_2$ may be the same or different groups selected from H, an alkyl, an aryl or an alkaryl group;
$-SO_2-$;
-O-;
$-NR_3-$ wherein $R_3$ is an alkyl, aryl or an alkaryl group;
and a $-C(CF_3)_2-$ group.

3. A process according to claim 1 or 2 wherein the carbonate or bicarbonate is present in an amount of at least 5%w/w of the bisamic acid to be cyclodehydrated.

4. A process according to any one of the preceding claims wherein the carbonate or bicarbonate is present in an anhydrous form.

5. A process according to any one of the preceding claims wherein the base component is sodium carbonate or potassium carbonate.

6. A process according to claim 3, 4 or 5 wherein the carbonate or bicarbonate is present in an amount of from 8-15%w/w based on the bisamic acid to be cyclodehydrated.

7. A process according to any one of the preceding claims wherein the cyclodehydration step is carried out in the presence of a catalytic amount of an inorganic salt selected from the acetate, chloride, bromide, nitrate or sulphate salt of potassium; sodium chloride; the chloride or sulphate salt of magnesium; cobalt acetate; and ferric sulphate.

8. A process according to any one of the preceding claims wherein the mole ratio of acetic anhydride to the bisamic acid to be cyclodehydrated is from 2-4 moles of anhydride per mole of bisamic acid.

9. A process according to any one of the preceding claims wherein the cyclodehydration is carried out in the presence of a polar, aprotic solvent in which both the reactants and base components are substantially soluble.

10. A process according to claim 9 wherein the solvent is dimethyl sulphoxide, formamide or N-methyl-pyrrolidone.

11. A process according to any one of the preceding claims wherein the cyclodehydration reactions is carried out at a temperature from 20-60 °C.

European Patent Office

# EUROPEAN SEARCH REPORT

Application number

EP 89311339.9

## DOCUMENTS CONSIDERED TO BE RELEVANT

| Category | Citation of document with indication, where appropriate, of relevant passages | Relevant to claim | CLASSIFICATION OF THE APPLICATION (Int. Cl. 5) |
|---|---|---|---|
| A | DE - A1 - 2 719 903 (E.I. DU PONT DE NEMOURS AND CO.) * Claims 1-3,13 * -- | 1,2,7, 11 | C 07 D 207/44<br>C 07 D 403/10 |
| A | DE - B - 2 040 094 (RHONE-POULENC S.A.) * Columns 1,2 * -- | 1,2,9 | |
| D,A | DE - A - 2 127 025 (TECHNOCHEMIE GMBH VERFAHRENS-TECHNIK) * Totality * -- | 1,2 | |
| D,A | US - A - 4 154 737 (GUS G. ORPHANIDES) * Abstract; column 1 * ---- | 1,2,7, 11 | |
| | | | **TECHNICAL FIELDS SEARCHED (Int. Cl. 5)** |
| | | | C 07 D 207/00 |

The present search report has been drawn up for all claims

| Place of search | Date of completion of the search | Examiner |
|---|---|---|
| VIENNA | 12-01-1990 | HEIN |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO Form 1503. 03.82